# EUROPEAN PATENT SPECIFICATION

(11) **EP 1 093 819 B1**
(45) Date of publication and mention of the grant of the patent: **03.05.2006**
(21) Application number: 00122704.0
(22) Date of filing: 06.02.1998
(51) Int. Cl.: A61K 38/29, A61K 47/18, C07C 229/00, C07C 233/00

(54) **Compound and composition for delivering active agents**
Verbindung und Arzneizusammensetzung zur Verabreichung von Wirkstoffen
Composé et composition pour l'administration d'agents actifs

(30) Priority: 07.02.1997 US 796336; 07.02.1997 US 796340; 07.02.1997 US 796338; 07.02.1997 US 797813; 07.02.1997 US 797816; 07.02.1997 US 797820; 07.02.1997 US 797100; 07.02.1997 US 796337; 07.02.1997 US 796334; 07.02.1997 US 796341; 07.02.1997 US 796339; 07.02.1997 US 797817; 07.02.1997 US 796335
(43) Date of publication of application: 25.04.2001
(62) Divisional of application: 98905042.2
(73) Proprietor: Emisphere Technologies, Inc., Tarrytown, New York 10591-6715 (US)
(72) Inventor: Leone-Bay, Andrea, Ridgefield, Connecticut 06877 (US); Ho, Koc-Kan, Monmouth Junction, New Jersey 08852 (US); Leipold, Harry R., Elmsford, New York 10523 (US); Milstein, Sam J., Larchmont, New York 20538 (US); Sarrubi, Donald J., Bronxville, New York 10708 (US); Wang, Eric, Ellicott City, Maryland 21042 (US); Gschneider, David, Stamford, Connecticut 06907 (US)
(74) Representative: Grünecker, Kinkeldey, Stockmair & Schwanhäusser Anwaltssozietät

(56) References cited:
- EP-A- 0 576 941
- WO-A-96/12473
- WO-A-97/36480

## Description

The present invention relates to a compound for delivering active agents, and particularly biologically or chemically active agents. This compound is used as carrier to facilitate the delivery of a cargo to a target. The carrier compound is well suited to form non-covalent mixtures with biologically-active agents for oral administration to animals.

### BACKGROUND OF THE INVENTION

Conventional means for delivering active agents are often severely limited by biological, chemical, and physical barriers. Typically, these barriers are imposed by the environment through which delivery occurs, the environment of the target for delivery, or the target itself. Biologically or chemically active agents are particularly vulnerable to such barriers.

For example in the delivery to animals of biologically active or chemically active pharmacological and therapeutic agents, barriers are imposed by the body. Examples of physical barriers are the skin and various organ membranes that must be traversed before reaching a target. Chemical barriers include, but are not limited to, pH variations, lipid bi-layers, and degrading enzymes.

These barriers are of particular significance in the design of oral delivery systems. Oral delivery of many biologically or chemically active agents would be the route of choice for administration to animals if not for biological, chemical, and physical barriers such as varying pH in the gastro-intestinal (Gl) tract, powerful digestive enzymes, and active agent impermeable gastro-intestinal membranes. Among the numerous agents which are not typically amenable to oral administration are biologically or chemically active peptides, such as calcitonin and insulin; polysaccharides, and in particular mucopolysaccharides including, but not limited to, heparin; heparinoids; antibiotics; and other organic substances. These agents are rapidly rendered ineffective or are destroyed in the gastro-intestinal tract by acid hydrolysis, enzymes, or the like.

Earlier methods for orally administering vulnerable pharmacological agents have relied on the co-administration of adjuvants (e.g., resorcinols and non-ionic surfactants such as polyoxyethylene oleyl ether and n-hexadecylpolyethylene ether) to increase artificially the permeability of the intestinal walls, as well as the co-administration of enzymatic inhibitors (e.g., pancreatic trypsin inhibitors, diisopropylfluorophosphate (DFF) and trasylol) to inhibit enzymatic degradation.

Liposomes have also been described as drug delivery systems for insulin and heparin. See, for example, U.S. Patent No. 4,239,754; Patel et al. (1976), *FEBS Letters,* Vol. 62, pg. 60; and Hashimoto et al. (1979), *Endocrinology Japan,* Vol. 26, pg. 337.

However, broad spectrum use of such drug delivery systems is precluded because: (1) the systems require toxic amounts of adjuvants or inhibitors; (2) suitable low molecular weight cargos, i.e. active agents, are not available; (3) the systems exhibit poor stability and inadequate shelf life; (4) the systems are difficult to manufacture; (5) the systems fail to protect the active agent (cargo); (6) the systems adversely alter the active agent; or (7) the systems fail to allow or promote absorption of the active agent.

More recently, microspheres of artificial polymers of mixed amino acids (proteinoids) have been used to deliver pharmaceuticals. For example, U.S. Patent No. 4,925,673 describes drug-containing proteinoid microsphere carriers as well as methods for their preparation and use. These proteinoid microspheres are useful for the delivery of a number of active agents.

WO 96/12473 discloses compounds and compositions for delivering agents.

These still is a need in the art for simple, inexpensive deliver, agents. Therefore the present inventions provides the compound of claim 10 and the pharmaceutical composition of claim 1. Preferred embodiments as defined in claims 2 to 9 and 11 to 13.

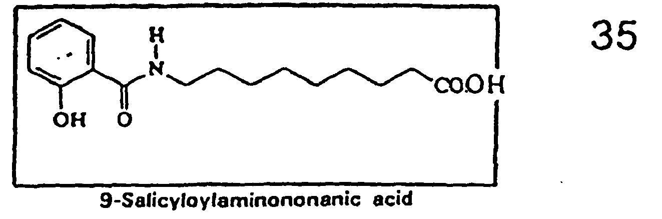

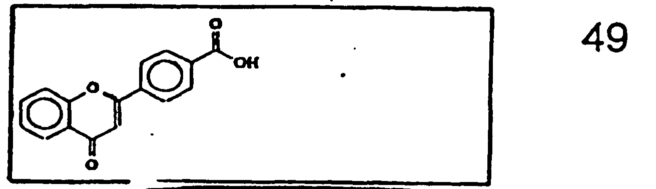

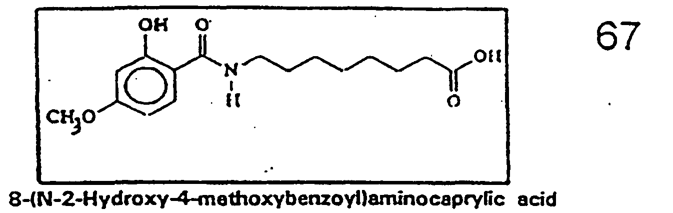

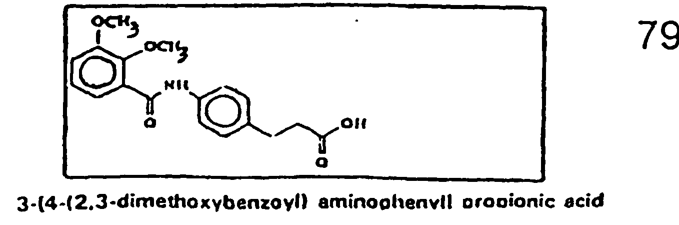

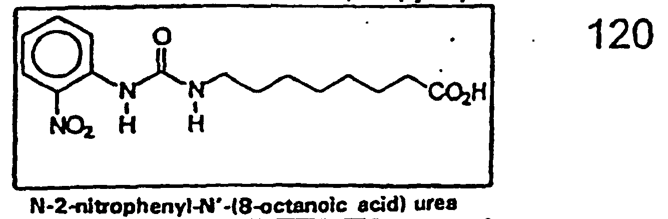

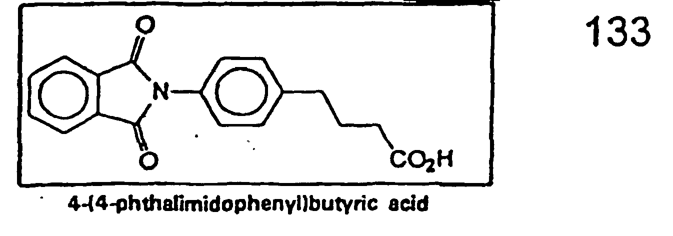

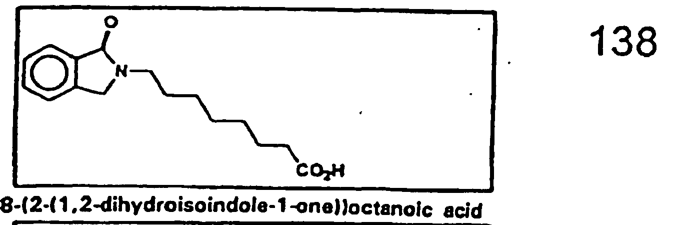

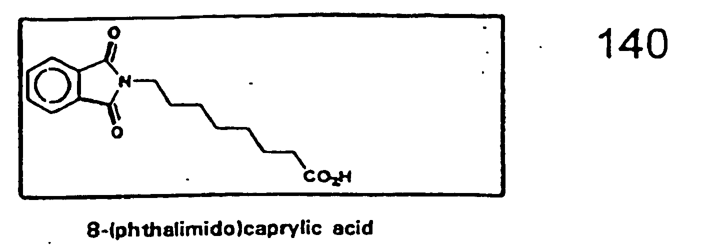

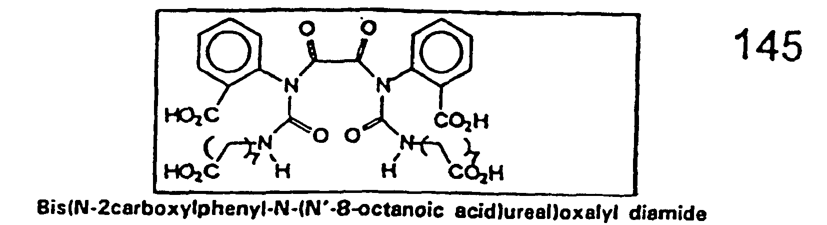

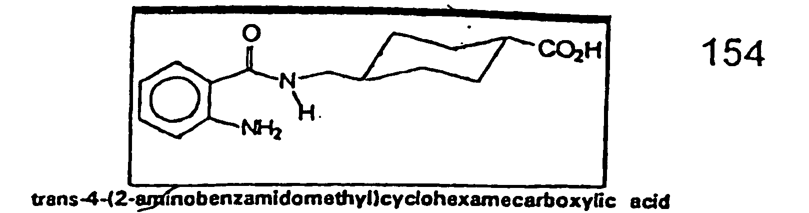

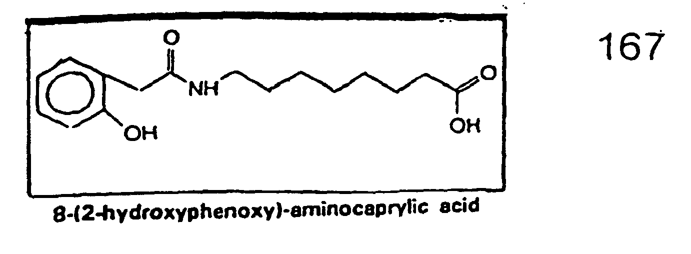

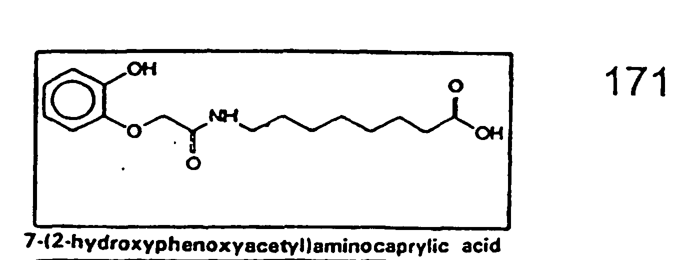

Compositions comprising the carrier compound as defined in the claim, and active agents are effective in delivering active agents to selected biological systems.

### DETAILED DESCRIPTION OF THE INVENTION

The specific compositions of the present invention include an active agent and a carrier. These compositions may be used to deliver various active agents through various biological, chemical, and physical barriers and are particularly suited for delivering active agents which are subject to environmental degradation. The compositions of the subject invention are particularly useful for delivering or administering biologically or chemically active agents to any animals such as birds including, but not limited to, chickens; mammals, such as primates and particularly humans; and insects.

Other advantages of the present invention include the use of easy to prepare, inexpensive raw materials. The compositions and the formulation methods of the present invention are cost effective, simple to perform, and amenable to industrial scale up for commercial production.

Subcutaneous, sublingual, and intranasal coadministration of an active agent, such as, for example, recombinant human growth hormone (rhGH); salmon calcitonin; heparin, including, but not limited to, low molecular weight heparin; parathyroid hormone; and compounds in compositions as described herein result in an increased bioavailability of the active agent compared to administration of the active agent alone.

### Active Agents

Active agents suitable for use in the present invention include biologically or chemically active agents, chemically active agents, including, but not limited to, fragrances, as well as other active agents such as, for example, cosmetics.

Biologically or chemically active agents include, but are not limited to, pesticides, pharmacological agents, and therapeutic agents. For example, biologically or chemically active agents suitable for use in the present invention include, but are not limited to, peptides, and particularly small peptides; hormones, and particularly hormones which by themselves do not or only a fraction of the administered dose passes through the gastro-intestinal mucosa and/or are susceptible to chemical cleavage by acids and enzymes in the gastro-intestinal tract; polysaccharides, and particularly mixtures of muco-polysaccharides; carbohydrates; lipids; or any combination thereof. Further examples include, but are not limited to, human growth hormones; bovine growth hormones; growth releasing hormones; interferons; interleukin-1; insulin; heparin, and particularly low molecular weight heparin; calcitonin; erythropoietin; atrial naturetic factor; antigens; monoclonal antibodies; somatostatin; adrenocorticotropin, gonadotropin releasing hormone; oxytocin; vasopressin; cromolyn sodium (sodium or disodium chromoglycate); vancomycin; desferrioxamine (DFO); parathyroid hormone anti-microbials, including, but not limited to anti-fungal agents; or any combination thereof.

Carrier Properties
Compound 67; Anal. Calculated for (C:62.12; H:7.49; N:4.53)
Found (C:61.94; H:7.45; N:4.43)
multing point 98-99°C

This carrier compound may be used to deliver active agents including, but not limited to, biologically or chemically active agents such as for example, pharmacological and therapeutic agents.

Salts such as, for example, sodium salt of these carrier compounds can be used as well.

The compound of the present invention can be readily prepared from amino acids.

For example, the compound may be prepared by reacting the single acid with the appropriate agent which reacts with free amino moiety present in the amino acids to form amides. Protecting groups may be used to avoid unwanted side reactions as would be known to those skilled in the art.

The carrier compound may be purified by recrystallization or by fractionation on solid column supports. Suitable recrystallization solvent systems include acetonitrile, methanol and tetrahydrofuran. Fractionation may be performed on a suitable solid column supports such as alumina, using methanol/n-propanol mixtures as the mobile phase; reverse phase column supports using trifluoroacetic acid/acetonitrile mixtures as the mobile phase; and ion exchange chromatography using water as the mobile phase. When anion exchange chromatography is performed, preferably a subsequent 0-500 mM sodium chloride gradient is employed.

### Delivery Systems

The compositions of the present invention may include one or more active agents.

In one embodiment, compound 67 or salts thereof may be used directly as a delivery carrier by simply mixing the compound or salt, with the active agent prior to administration.

The administration mixtures are prepared by mixing an aqueous solution of the carrier with an aqueous solution of the active ingredient, just prior to administration. Alternatively, the carrier and the biologically or chemically active ingredient can be admixed during the manufacturing process. The solutions may optionally contain additives such as phosphate buffer salts, citric acid, acetic acid, gelatin, and gum acacia.

Stabilizing additives may be incorporated into the carrier solution. With some drugs, the presence of such additives promotes the stability and dispersibility of the agent in solution.

The stabilizing additives may be employed at a concentration ranging between about 0.1 and 5 % (W/V), preferably about 0.5 % (W/V). Suitable, but non-limiting, examples of stabilizing additives include gum acacia, gelatin, methyl cellulose, polyethylene glycol, carboxylic acids and salts thereof, and polylysine. The preferred stabilizing additives are gum acacia, gelatin and methyl cellulose.

The amount of active agent is an amount effective to accomplish the purpose of the particular active agent. The amount in the composition typically is a pharmacologically, biologically, therapeutically, or chemically effective amount. However, the amount can be less than a pharmacologically, biologically, therapeutically, or chemically effective amount when the composition is used in a dosage unit form, such as a capsule, a tablet or a liquid, because the dosage unit form may contain a multiplicity of carrier/biologically or chemically active agent compositions or may contain a divided pharmacologically, biologically, therapeutically, or chemically effective amount. The total effective amounts can then be administered in cumulative units containing, in total, pharmacologically, biologically, therapeutically or chemically active amounts of biologically or pharmacologically active agent.

The total amount of active agent, and particularly biologically or chemically active agent, to be used can be determined by those skilled in the art. However, it has surprisingly been found that with some biologically or chemically active agents, the use of the presently disclosed carriers provides extremely efficient delivery, particularly in oral, intranasal, sublingual, intraduodenal, rectal, vaginal, buccal, ophthalmic, or subcutaneous systems as well as systems for crossing the blood/brain barrier. Therefore, lower amounts of biologically or chemically active agent than those used in prior dosage unit forms or delivery systems can be administered to the subject, while still achieving the same blood levels and therapeutic effects.

The amount of carrier in the present composition is a delivery effective amount and can be determined for any particular carrier or biologically or chemically active agent by methods known to those skilled in the art.

Dosage unit forms can also include any of excipients; diluents; disintegrants; lubricants; plasticizers; colorants; and dosing vehicles, including, but not limited to water, 1,2-propane diol, ethanol, olive oil, or any combination thereof.

Administration of the present compositions or dosage unit forms preferably is oral or by intraduodenal injection.

The delivery compositions of the present invention may also include one or more enzyme inhibitors. Such enzyme inhibitors include, but are not limited to, compounds such as actinonin or epiactinonin and derivatives thereof. These compounds have the formulas below: 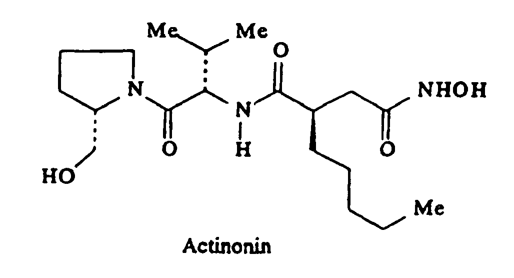 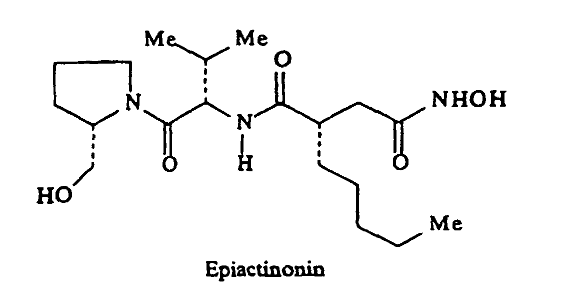 Derivatives of these compounds are disclosed in U.S. Patent No. 5,206,384. Actinonin derivatives have the formula: 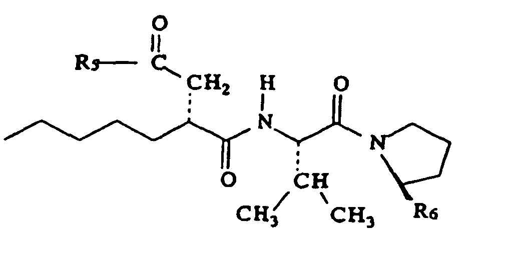 wherein R⁵ is sulfoxymethyl or carboxyl or a substituted carboxy group selected from carboxamide, hydroxyaminocarbonyl and alkoxycarbonyl groups; and R⁶ is hydroxyl, alkoxy, hydroxyamino or sulfoxyamino group. Other enzyme inhibitors include, but are not limited to, aprotinin (Trasylol) and Bowman-Birk inhibitor.

The compound and compositions of the subject invention are useful for administering biologically or chemically active agents to any animals such as birds; mammals, such as primates and particularly humans; and insects. The system is particularly advantageous for delivering chemically or biologically or chemically active agents which would otherwise be destroyed or rendered less effective by conditions encountered before the active agent its target zone (i.e. the area in which the active agent of the delivery composition are to be released) and within the body of the animal to which they are administered. Particularly, the compounds and compositions of the present invention are useful in orally administering active agents, especially those which are not ordinarily orally deliverable.

### DESCRIPTION OF THE PREFERRED EMBODIMENTS

The following examples illustrate the invention without limitation. All parts are given by weight unless otherwise indicated.

### Example 1 - Carrier Preparation

**General Preparations of Carriers.** The following procedures were used to prepare the reference compounds described herein. Many of the compounds were prepared by reaction of the appropriate amino acid with the appropriate acid chloride. The preparation of compound 79 is given as a representative example of the compounds prepared in this manner.

**Preparation of Compound 79. Method A.** A 1 L round bottom flask fitted with a magnetic stirrer was charged with 3-(4-aminophenyl)propionic acid (46.3 g, 0.28 moles, 1.17 equiv.) and 2 M aqueous sodium hydroxide (300 mL). 2,3-dimethoxybenzoylchloride (48.0 g, 0.24 moles, 1.00 equiv.) was added portionwise over 1 h to the stirred solution. After the addition, the reaction was stirred for 2.5 h at ambient temperature, and the pH of the solution was kept at ca 10 by the addition of 10 M sodium hydroxide. The solution was then acidified with 1 M hydrochloric acid (3 x 100 mL), water (100 mL), and air dried. It was redissolved in boiling acetone (*ca* 500 mL), decolorized with activated charcoal (3g), and filtered. Water (1.5 L) was added to the filtrate to induce the formation of a brown oil. The brown oil solidified upon stirring at room temperature for 10 min. The crude solid was collected by filtration and recrystallized from 70% methanol-water (v/v) to afford compound 79 as a tan solid (39.5) g, 50%).

**Preparation of Compound 79. Method B.** A 2 L three-neck round bottom flask was fitted with a magnetic stirrer and two addition funnels under an argon atmosphere. A suspension of 3-(4-aminophenyl)propionic acid (46.3 g, 0.28 moles, 1.17 equiv.) in ethyl acetate (700 mL) was added to the flask. A solution of 2,3-dimethoxybenzoylchloride (48.0 g, 0.24 moles, 1.00 equiv.) in ethyl acetate (250 mL) was charged to one of the addition funnels and added dropwise over 1 h. Triethylamine (28.20 g, 0.28 moles, 1.00 equiv.) was subsequently charged to the second funnel and added dropwise over 15 min. The reaction was stirred at ambient temperature for 3 h, and the solvent was evaporated *in vacuo* giving a residual brown oil. Water (600 mL) was added to the residue followed by sodium hydroxide (2 M, 500 mL), and the mixture was stirred at ambient temperature for 3 hours. The resultant brown solution was acidified with 2 M hydrochloric acid (*ca* 1 L). After cooling the mixture in an ice bath for 1 h, a yellow solid formed and was collected by filtration. The solid was washed with water (3 x 1.5 L) and recrystallized from 50% ethanol-water (v/v) to give compound 79 as a tan solid (59.2 g, 68%).

**Preparation of Compound 79. Method C.** A 2 L round bottom flask equipped with a magnetic stirrer and a reflux condenser was charged with a suspension of 3-(4-aminophenyl)propionic acid (46.3 g, 0.28 moles, 1.17 equiv.) in dichloromethane (560 mL). Chlorotrimethylsilane (62.36 g, 0.57 moles, 2.05 equiv.) was added in one portion, and the mixture was heated to reflux for 1 h under argon. The reaction was allowed to cool to room temperature and was placed in an ice bath (internal temperature <10°C). The reflux condenser was replaced with an addition funnel containing triethylamine (42.50 g, 0.42 moles, 1.50 equiv.). The triethylamine was added dropwise over 15 min, and a yellow solid formed during the addition. The funnel was replaced by another addition funnel containing a solution of 2,3-dimethoxybenzoylchloride (48.0 g, 0.24 moles, 1.00 equiv. in dichloromethane (100 mL). The solution was added dropwise over 30 min. The reaction was stirred in the ice bath for another 30 min and at ambient temperature for 1 h. The dichloromethane was evaporated *in vacuo* to give a brown oil. The brown oil was cooled in an ice bath, and an ice-cold solution of 2 M sodium hydroxide (700 mL) was added. The ice bath was removed, and the reaction was stirred for 2 h to afford a clear brown solution. The solution was acidified with 2 M sulfuric acid (400 mL) and stored at *ca* 5°C for 1 hour. A yellow solid formed and was collected by filtration. The solid was washed with water (3 x 100 mL) and recrystallized from 50% ethanol-water (v/v) to afford compound 79 as tan needles (64.7 g, 82%).

Compound 67, was also prepared by this process.

**Preparation of Compound 35.** A solution of *O*-acetylsalicyloyl chloride (24.68 g, 124 mmol, 1 equiv) in tetrahydrofuran (300 mL) was cooled in an ice bath. Triethylamine (25 g, 249 mmol, 2 equiv) was added dropwise via an additional funnel. The methyl 9-aminononanoate hydrochloride was dissolved in DMF (190 mL, slightly warm to dissolve), charged to an addition funnel and added dropwise to the above mixture. The reaction was stirred in the ice-bath for 20 min and at room temperature for 2 h. Evaporation of the THF under reduced pressure gave a pink DMF solution. The pink solution was cooled in an ice-bath, and 2 M aqueous sodium hydroxide (300 mL) was added. After being stirred at room temperature for 12 h, the mixture was acidified with 2 M hydrochloric acid (500 mL). The solution was cooled in an ice-bath, and a solid formed. The solid was collected by filtration and was recrystallized from 50% ethanol/water to give compound 35 (32 g, 87%) as an off-white solid.

**Preparation of Compound 49**. 1-(2-hydroxyphenyl)-3-(4-methyl benzoate)-1,3-propane dione (3.00 g, 0.0101 mil.) is placed in a 100 ml round bottomed flask fitted with argon purge, magnetic stir bar and cold water condenser. Glacial acetic acid (20 mls) and concentrated sulfuric acid (5 mls) were added, and heating of the reaction mixture was initiated. The reaction mixture was allowed to heat at reflux for 6 h before heating was discontinued. The reaction mixture was allowed to come to room temperature, and then was poured into 100 mls of ice/water. This was stirred for approximately 1/2 h before the mixture was filtered, and a brown solid was isolated. The brown solid was recrystallized twice from acetic acid, yielding compound 49 as a tan solid (1.44 g, 53.8%).

**Preparation of Compound 167**. 2-coumaranone (4.21 g, 0.0314 mol) was dissolved, with stirring, in acetonitrile (75 mls) in a 250 ml round bottomed flask fitted with a magnetic stir bar, argon purge and cold water condenser. Triethylamine (3.18 g, 0.0314 mol) and 8-aminocaprylic acid (5.00 g, 0.0314 mol) were added, and a tan slurry was formed. Heating was started, and the reaction mixture was allowed to reflux overnight. After heating overnight, thin layer chromatography of the reaction mixture (50% ethyl acetate / 50% hexane) indicated that the reaction had gone to completion. Heating was stopped, the reaction mixture was allowed to cool to room temperature, and was concentrated *in vacuo.* The resulting residue was taken up in methylene chloride, and was washed with two, 100 ml portions of 1 N hydrochloric acid solution. The methylene chloride layer was dried with sodium sulfate and was concentrated *in vacuo.* The resulting tan solid was allowed to dry *in vacuo* overnight, yielding compound 167 as a tan solid (8.35 g, 70.4%).

**Preparation of Compound 171**. 1,4-benzodioxan-2-one (3.93 g, 0.0262 mol) was dissolved, with stirring, in acetonitrile (70 mls) in a 250 ml round bottomed flask fitted with a magnetic stir bar, argon purge and cold water condenser. Triethylamine (2.64 g, 0.0262 mol) and 8-aminocaprylic acid (500 g, 0.0262 mol) were added and a tan slurry was formed. Heating was started, and the reaction mixture was allowed to reflux for approximately 3 hours. At this time, thin layer chromatography of the reaction mixture (50% ethyl acetate /50% hexane) indicated that the reaction had gone to completion. Heating was discontinued, and the reaction mixture was allowed to cool to room temperature and was concentrated *in vacuo.* The resulting residue was taken up in methylene chloride and was washed with a 100 ml portion of 1N hydrochloric acid solution. At this time, a tan solid was noted to precipitate, and it was isolated by filtration. This tan solid was washed further with an additional 100 ml portion of 1 N hydrochloric acid solution, and then with 100 ml of water. The resulting tan solid was allowed to dry *in vacuo* overnight yielding Compound 171 as a tan solid (7.73 g, 95.6%).

**Preparation of Compound 120**. A solution of 3.00 g (18.3 mmol) of 2-nitrophenylisocyanate and 5 mL of tetrahydrofuran was dropwise over 10 min to an ice bath-cooled solution of 2.08 g (13.1 mmol) of 8-aminocaprylic acid, 1.40 mL of 10 N NaOH and 40 mL of water. The reaction mixture was stirred an additional 30 min, warmed to 25°C and treated with 3% HCI solution until the pH was 5. The yellow precipitate was filtered off and rinsed with 100 ml of water. The yellow solid was recrystallized in 2-propanol and water to give 3.7 g of compound 120 as pale yellow crystals.

**Preparation of Compound 133.** A suspension of 2.40 g (16.3 mmol) and 2.80 g (15.6 mmol) of 4-(4aminophenyl)butyric acid in 20 mL of propylene glycol, 2.40 mL (1.74 g, 17.3 mmol) of triethylamine and 10 mg (0.08 mmol) of dimethylaminopyridine was heated to 140°C. The mixture became a clear solution after 5 min at 140°C. After stirring for 330 min, the reaction mixture was cooled to 25°C and diluted with 20 mL of water. The solid phthalimide which had formed was filtered off. The filtrate was acidified with 3% HCI solution. The resulting solid was filtered off and was recrystallized from 2-propanol and water to give 0.62 g of compound 133 as a tan solid.

**Preparation of Compound 138**. A solution of 1.73 g (12.9 mmol) of phthalic dialdehyde, 2.04 g 8-aminocaprylic acid and 20 mL of acetic acid was heated to reflux for 10 min. The reaction mixture was cooled to 40°C, diluted with water and extracted with CH₂Cl₂ (2 X 20 mL). The organic phase was washed with water and brine, dried over Na₂SO₄ and evaporated. The residue was dissolved in ether and extracted with 2N NaOH. The layers were separated. The aqueous layer was made acidic with 3% HCI and extracted with CH₂Cl₂. The organic phase was dried over Na₂SO₄ and evaporated. The yellow residue was crystallized from acetonitrile and water to give 1.25 g of compound 138 as a yellow solid.

**Preparation of Compound 140**. A mixture of 1.40 g (9.48 mmol) of phthalic anhydride and 1.51 g (9.48 mmol) of 8-aminocaprylic acid was heated to 150°C for 5 min. Upon cooling, 2.61 g of solid compound 140 was received.

**Preparation of Compound 145**. A suspension of 2.11 g (10.1 mmol) ethyl carbamoylanthranilic acid and 5 mL of CH₂Cl₂ was treated with 2.20 mL of oxalyl chloride. After stirring for 1 h the volatiles were stripped off. At that same time, a suspension of 1.60 g (10.1 mmol) of 8-aminocaprylic acid and 15 mL of CH₂Cl₂ was treated with 2.60 mL (2.23 g, 20.5 mmol) of TMSCI. This mixture was heated to reflux for 90 min, cooled in an ice bath and treated with 4.30 mL (3.12 g, 30.9 mmol) of triethylamine. Five min later, a slurry of the residue from the oxalyl chloride reaction in 20 mL of CH₂Cl₂ was added. The reaction mixture was warmed to 25°C and stirred overnight. Upon acidification of the mixture with 3% HCI, a white solid formed. The solid was filtered off and recrystallized from EtOH and water to give 1.88 g of compound 145.

**Preparation of Compound 154**. A suspension of 4.02 g(25.6 mmol) of trans-4-aminomethylcyclohexane-carboxylic acid, 4.18 g (25.6 mmol) of isatoic anhydride, 20 mL of CH₂Cl₂, 20 mL of dioxane, and 4 mL of water was heated to reflux for 12 h. The solution was cooled to 25°C and extracted with ether (4 x 20 mL). The organic layer was dried over Na₂SO₄ and concentrated. The resulting solid was recrystallized from EtOH and water to give 4.95 g of compound 154.

### Example 4 - Recombinant Human Growth Hormone Dosing Solutions

Intracolonic dosing compositions containing 25 mg/kg of carrier and 1 mg/kg of rHGH in phosphate buffer or oral gavage dosing solutions containing 600 mg/kg of carrier and 3 mg/kg of rHGH in phosphate buffer were prepared with carrier 67.

The dosing solutions are designated R- carrier number - DS.

### Comparative Example 4A - Recombinant Human Growth Hormone Dosing Solutions

An intracolonic dosing solution was prepared according to the procedure of Example 4, substituting a carrier having the formula 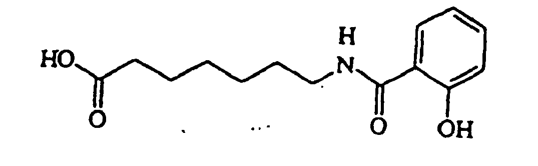 for the carrier. This dosing solution is designated as R-35A-DS.

### Comparative Example 4B - Recombinant Human Growth Hormone Dosing Solutions

An intracolonic dosing solution was prepared according to the procedure of Example 4, substituting a carrier having the formula 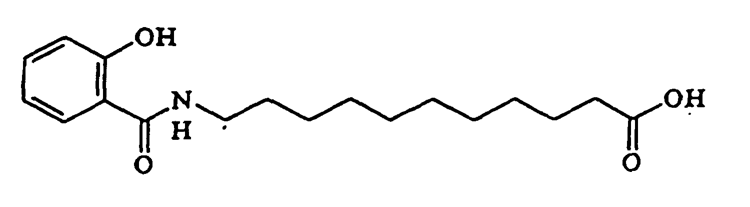 for the carrier. This dosing solution is designated as R-358-DS.

### Comparative Example 4C - Recombinant Human Growth Hormone Dosing Solutions

An intracolonic dosing solution was prepared according to the procedure of Example 4, substituting a carrier having the formula 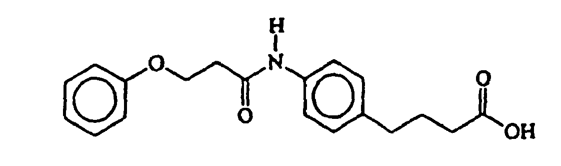 for the carrier. This dosing solution is designated as R-9A-DS.

### Example 5 - In Vivo Recombinant Human Growth Hormone Delivery

Male Sprague-Dawley rats weighing 200-250g were fasted for 24 hours and administered ketamine (44 mg/kg) and chlorpromazine (1.5 mg/kg) 15 minutes prior to dosing. The rats were administered one of the dosing solutions of Example 4 by either oral gavage or intracolonic instillation. Blood samples were collected serially from the tail artery for determination of serum rHGH concentrations. Serum rHGH concentrations were quantified by an rHGH immunoassay test kit.

Results are illustrated in Table 3, below.

### Comparative Example 5A - In Vivo Recombinant Human Growth Hormone Delivery

The procedure of Example 5 was followed, substituting the dosing solutions of Comparative Examples 4A-4C for the dosing solutions.

Results are illustrated in Table 3, below.

### Comparative Example 5B - In Vivo Recombinant Human Growth Hormone Delivery

The procedure of Example 5 was followed, with dosing solutions of active agent (at a dose of 1 mg of rHGH/kg (intracolonic) or 3 mg of rHGH/kg (oral) and no carrier. These dosing solutions are designated R-ØD-DS and R-ØE-DS, respectively. Results are illustrated in Table 3, below.

| **TABLE 3 - *In Vivo* Recombinant Human Growth Hormone Delivery** | |
|---|---|
| Dosing Solution | Mean Peak Serum [rHGH] ± Standard Deviation (ng/ml) |
| | 19 ± 22 (PO) |
| R-67-DS | 88 ± 24 (IC) |
| R-35A-DS | 17 ± 3 (IC) |
| R-35B-DS | 42 ± 28 (IC) |
| R-9A-DS | 55 ± 17 (IC) |
| R-ØD-DS | 0 ± 0 (IC) |
| R-ØE-DS | 0 ± 0 (IC) |

### Example 7 - Heparin Dosing Solutions

Intracolonic dosing compositions containing 50 mg/kg of carrier and 25 mg/kg of heparin in 25% aqueous propylene glycol or oral gavage dosing solutions containing 300 mg/kg of carrier and 100 mg/kg of heparin in 25% aqueous propylene glycol were prepared with carrier 67. The dosing solutions are designated H-carrier number-DS.

### Comparative Example 7A - Heparin Dosing Solutions

Comparative intracolonic dosing compositions were prepared according to the procedure of Example 7, substituting the following carriers for the carrier.

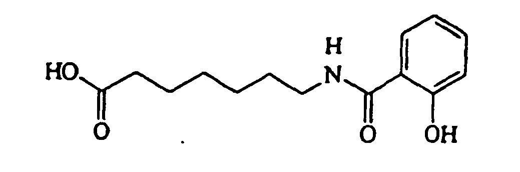

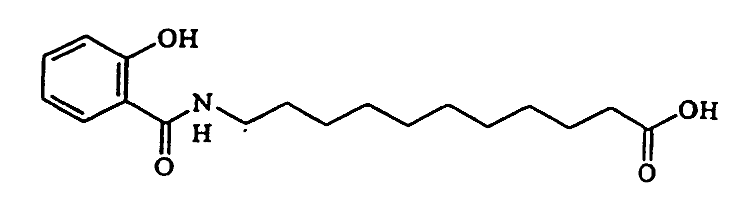

These dosing solutions are designated H-35A-DS, H-35B-DS, and H-109A-DS, respectively.

### Examples 8 - In Vivo Evaluation of Heparin in Rats

The dosing solutions of Example 7 were administered to fasted rats either by oral gavage or intracolonic instillation.

Blood samples were collected by cardiac puncture following the administration of ketamine (44 mg/kg). Heparin activity was determined by utilizing the activated partial thromboplastin time (APTT) according to the method of Henry, J.B., Clinical Diagnosis and Management by Laboratory Methods; Philadelphia, PA; W.B. Saunders (1979).

Results are in illustrated in Table 4, below.

### Comparative Examples 8A - In Vivo Evaluation of Heparin in Rats

The dosing solutions of Comparative Example 7A were administered to fasted rats by intracolonic instillation. Blood samples were collected and heparin activity was determined by the method of Example 8.

Results are illustrated in Table 4, below.

### Comparative Example 8B - In Vivo Evaluation of Heparin in Rats

An intracolonic dosing solution of 25 mg/kg of heparin and an oral gavage dosing solution of 100 mg/kg of heparin were administered to fasted rats. These dosage solutions were designated H-ØA-DS and H-ØB-DS, respectively.

Blood samples were collected, and heparin activity was determined by the methods of Example 8.

Results are illustrated in Table 4, below.

| **TABLE 4 - *In Vivo* Evaluation of Heparin in Rats** | |
|---|---|
| Dosing Solution | Heparin APTT (sec) |
| H-67-DS | 76 ± 36 (IC) |
| H-35A-DS | 61 ± 29 (IC) |
| H-358-DS | 51 ± 30 (IC) |
| H-169-DS | 23 ± 2 (IC) |
| H-ØA-DS | 23 ±2 (PO) |
| H-Ø8-DS | 33 ± 6 (IC) |

## Claims

1. A pharmaceutical composition or a unit dosage form comprising the pharmaceutical composition comprising:
(A) at least one active agent; and
(B) a compound having the formula 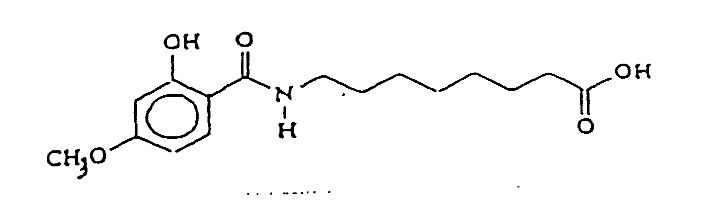
or a salt thereof.

2. The pharmaceutical composition or unit dosage form of claim 1, wherein the active agent is selected from the group consisting of biologically active agents, chemically active agents, and any combination thereof.

3. The pharmaceutical composition or unit dosage form of claim 2, wherein the biologically active agent comprises at least one peptide, mucopolysaccharide, carbohydrate, or lipid.

4. The pharmaceutical composition or unit dosage form of claim 2, wherein the biologically active agent is selected from the group consisting of human growth hormones, bovine growth hormones, growth hormone-releasing hormones, interferons, interleukin-1, interleukin-II, insulin, heparin, low molecular weight heparin, calcitonin, erythropoietin, atrial naturetic factor, antigens, monoclonal antibodies, somatostatin, adreno-corticotropin, gonadrotropin releasing hormone, oxytocin, vasopressin, cromolyn sodium, vancomycin, desferrioxamine, parathyroid hormone, and any combination thereof.

5. The pharmaceutical composition or unit dosage form of claim 4, wherein the biologically active agent comprises interferon, interleukin-1, interleukin-II, insulin, heparin, low molecular weight heparin, calcitonin, oxytocin, vasopressin, vancomycin, desferrioxamine, parathyroid hormone, or any combination thereof.

6. A dosage unit form comprising
(A) the pharmaceutical composition of any of claims 1 to 5; and
(B)
(a) an excipient,
(b) a diluent,
(c) a disintegrant,
(d) a lubricant,
(e) a plasticizer,
(f) a colorant,
(g) a dose vehicle, or
(h) any combination thereof.

7. The dosage unit form of claim 6, comprising a tablet, a capsule, or a liquid.

8. The dosage unit form of any of claims 6 and 7, wherein said dosing vehicle is selected from the group consisting of water, 1,2-propane diol, ethanol, olive oil and any combination thereof.

9. The pharmaceutical composition or unit dosage form as defined in claims 1 to 5 to be administered orally, intranasally, sublingually, intraduodenally, subcutaneously, rectally, vaginally, bucally or ophthalmically.

10. A compound having the formula 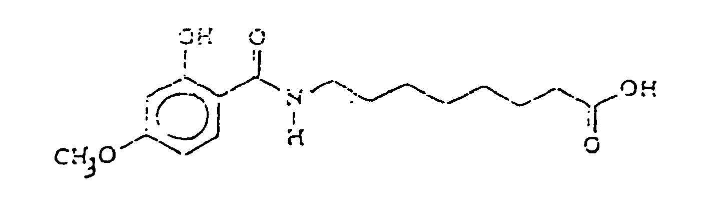 or a salt thereof.

11. A method for preparing a composition, said method comprising mixing:
(A) at least one active agent;
(B) the compound of claim 10; and
(C) optionally, a dosing vehicle.

12. The pharmaceutical composition of any of claims 1 to 5 capable of passing a biologically active agent across the blood/brain barrier of an animal.

13. The pharmaceutical composition according to claim 1, wherein the at least one active agent is human growth hormone.

## Patentansprüche

1. Pharmazeutische Zusammensetzung oder Einzeldarreichungsform, umfassend die pharmazeutische Zusammensetzung, umfassend:
(A) wenigstens einen Wirkstoff; und
(B) eine Verbindung mit der Formel 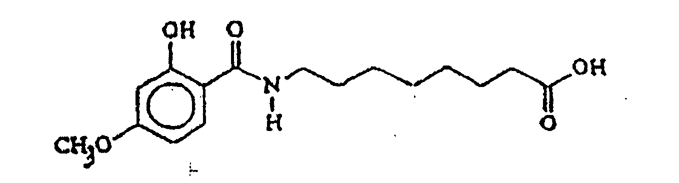
oder ein Salz davon.

2. Pharmazeutische Zusammensetzung oder Einzeldarreichungsform nach Anspruch 1, wobei der Wirkstoff ausgewählt ist aus der Gruppe bestehend aus biologischen Wirkstoffen, chemischen Wirkstoffen und einer beliebigen Kombination davon.

3. Pharmazeutische Zusammensetzung oder Einzeldarreichungsform nach Anspruch 2, wobei der biologische Wirkstoff wenigstens ein Peptid, Mucopolysaccharid, Kohlenhydrat oder Lipid umfasst.

4. Pharmazeutische Zusammensetzung oder Einzeldarreichungsform nach Anspruch 2, wobei der biologische Wirkstoff ausgewählt ist aus der Gruppe bestehend aus menschlichen Wachstumshormonen, Rinderwachstumshormonen, Wachstumshormon-freisetzenden Hormonen, Interferonen, Interteukin-1, Interleukin-II, Insulin, Heparin, Heparin mit niedrigem Molekulargewicht, Calcitonin, Erythropoetin, atrialem natriuretischem Faktor, Antigenen, monoklonalen Antikörpern, Somatostatin, Adrenokortikotropin, Gonadotropin-Releasing-Hormon, Oxytocin, Vasopressin, Cromolyn-Natrium, Vancomycin, Desferrioxamin, Parathormon und einer beliebigen Kombination davon.

5. Pharmazeutische Zusammensetzung oder Einzeldarreichungsform nach Anspruch 4, wobei der biologische Wirkstoff Interferon, Interleukin-1, Interleukin-II, Insulin, Heparin, Heparin mit niedrigem Molekulargewicht, Calcitonin, Oxytocin, Vasopressin, Vancomycin, Desferrioxamin, Parathormon oder eine beliebige Kombination davon umfasst.

6. Einzeldarreichungsform, umfassend:
(A) die pharmazeutische Zusammensetzung nach einem der Ansprüche 1 bis 5; und
(B)
(a) ein Excipiens,
(b) ein Verdünnungsmittel bzw. Streckmittel,
(c) ein Zerfallhilfsmittel,
(d) ein Schmiermittel,
(e) einen Weichmacher,
(f) ein Färbemittel,
(g) ein Dosiervehikel, oder
(h) eine beliebige Kombination davon.

7. Einzeldarreichungsform nach Anspruch 6, umfassend eine Tablette, eine Kapsel oder eine Flüssigkeit.

8. Einzeldarreichungsform nach einem der Ansprüche 6 und 7, wobei das Dosiervehikel ausgewählt ist aus der Gruppe bestehend aus Wasser, 1,2-Propandiol, Ethanol, Olivenöl und einer beliebigen Kombination davon.

9. Pharmazeutische Zusammensetzung oder Einzeldarreichungsform wie in den Ansprüchen 1 bis 5 definiert, welche oral, intranasal, sublingual, intraduodenal, subkutan, rektal, vaginal, bukkal oder ophthalmisch verabreicht werden soll.

10. Verbindung mit der Formel 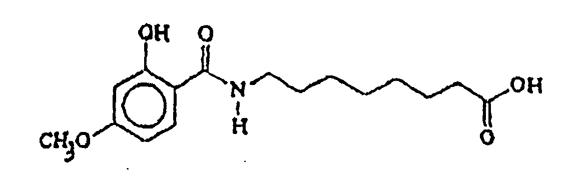 oder ein Salz davon.

11. Verfahren zum Herstellen einer Zusammensetzung, wobei das Verfahren das Vermischen von:
(A) wenigstens einem Wirkstoff;
(B) der Verbindung nach Anspruch 10; und
(C) gegebenenfalls einem Dosiervehikel
umfasst.

12. Pharmazeutische Zusammensetzung nach einem der Ansprüche 1 bis 5, die imstande ist, einen biologischen Wirkstoff über die Blut-Hirn-Schranke eines Tieres zu transportieren.

13. Pharmazeutische Zusammensetzung nach Anspruch 1, wobei der wenigstens eine Wirkstoff menschliches Wachstumshormon ist.

## Revendications

1. Composition pharmaceutique ou forme de dose unitaire comprenant la composition pharmaceutique comprenant :
(A) au moins un agent actif ; et
(B) un composé ayant la formule 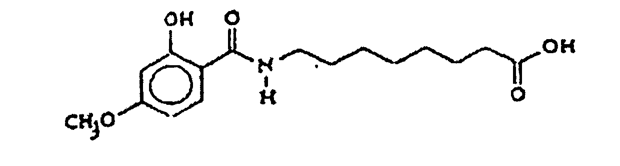
ou un sel de celui-ci.

2. Composition pharmaceutique ou forme de dose unitaire selon la revendication 1, dans laquelle l'agent actif est choisi dans le groupe constitué d'agents biologiquement actifs, agents chimiquement actifs et toute combinaison de ceux-ci.

3. Composition pharmaceutique ou forme de dose unitaire selon la revendication 2, dans laquelle l'agent biologiquement actif comprend au moins un peptide, mucopolysaccharide, glucide ou lipide.

4. Composition pharmaceutique ou forme de dose unitaire selon la revendication 2, dans laquelle l'agent biologiquement actif est choisi dans le groupe constitué des hormones de croissance humaines, hormones de croissance bovines, hormones de libération de l'hormone de croissance, interférons, interleukine-1, interleukine-II, insuline, héparine, héparine de faible poids moléculaire, calcitonine, érythropoïétine, facteur atrial natriurétique, antigènes, anticorps monoclonaux, somatostatine, adrénocorticotropine, hormone de libération de gonadotropine, ocytocine, vasopressine, acide cromoglycique, vancomycine, déféroxamine, parathormone et toute combinaison de ceux-ci.

5. Composition pharmaceutique ou forme de dose unitaire selon la revendication 4, dans laquelle l'agent biologiquement actif comprend l'interféron, interleukine-1, interleukine-II, insuline, héparine, héparine de faible poids moléculaire, calcitonine, ocytocine, vasopressine, vancomycine, déféroxamine, parathormone et toute combinaison de ceux-ci.

6. Forme de dose unitaire comprenant
(A) la composition pharmaceutique de l'une quelconque des revendications 1 à 5 ; et
(B)
(a) un excipient,
(b) un diluant,
(c) un désintégrant,
(d) un lubrifiant,
(e) un plastifiant,
(f) un colorant,
(g) un véhicule de dose, ou
(h) toute combinaison de ceux-ci.

7. Forme de dose unitaire selon la revendication 6, comprenant un comprimé, une capsule ou un liquide.

8. Forme de dose unitaire selon l'une quelconque des revendications 6 et 7, dans laquelle ledit véhicule de dose est choisi dans le groupe constitué de l'eau, 1,2-propane diol, éthanol, huile d'olive et toute combinaison de ceux-ci.

9. Composition pharmaceutique ou forme de dose unitaire telle que définie dans les revendications 1 à 5 destinée à être administrée par voie orale, intra-nasale, sublinguale, intraduodénale, sous-cutanée, rectale, vaginale, buccale ou ophtalmique.

10. Composé ayant la formule 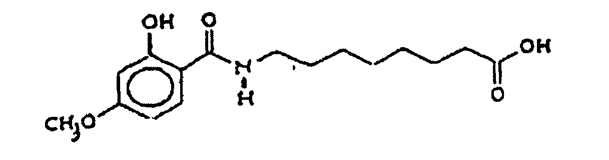 ou sel de celui-ci.

11. Procédé pour préparer une composition, ledit procédé comprenant le mélange :
(A) d'au moins un agent actif ;
(B) du composé selon la revendication 10 ; et
(C) éventuellement, d'un véhicule de dose.

12. Composition pharmaceutique selon l'une quelconque des revendications 1 à 5, capable de faire passer un agent biologiquement actif au travers de la barrière hémato-encéphalique d'un animal.

13. Composition pharmaceutique selon la revendication 1, dans laquelle le au moins un agent actif est l'hormone de croissance humaine.
